# EUROPEAN PATENT SPECIFICATION

(11) **EP 3 294 128 B1**
(45) Date of publication and mention of the grant of the patent: **29.06.2022**
(21) Application number: 16792961.1
(22) Date of filing: 10.05.2016
(51) Int. Cl.: A61B 5/0538, A61B 5/00, A61B 5/145, A61B 5/1486

(54) **BIOSENSOR ELECTRODE STRUCTURE AND BIOSENSOR INCLUDING THE SAME**
BIOSENSORELEKTRODENSTRUKTUR UND BIOSENSOR DAMIT
STRUCTURE D'ÉLECTRODE DE CAPTEUR BIOLOGIQUE, ET CAPTEUR BIOLOGIQUE LA COMPRENANT

(30) Priority: 11.05.2015 US 201562159565 P; 02.10.2015 KR 20150139111
(43) Date of publication of application: 21.03.2018
(73) Proprietor: Samsung Electronics Co., Ltd., Gyeonggi-do 16677 (KR)
(72) Inventor: OH, Young-jae, Suwon-si Gyeonggi-do 16509 (KR); KIM, Kwang-bok, Incheon 21406 (KR); CHO, Seong-je, Suwon-si Gyeonggi-do 16538 (KR); CHO, Jae-geol, Yongin-si Gyeonggi-do 16923 (KR); CHOI, Hyoung-seon, Seoul 04385 (KR); JUNG, Sun-tae, Yongin-si Gyeonggi-do 16923 (KR); CHO, Chul-ho, Yongin-si Gyeonggi-do 16831 (KR)
(74) Representative: HGF
(86) International application number: PCT/KR2016/004872
(87) International publication number: WO 2016/182315

(56) References cited:
- US-A1- 2002 072 054
- US-A1- 2008 214 918
- US-A1- 2009 099 427
- US-A1- 2011 077 490
- US-A1- 2011 144 466
- US-A1- 2013 197 333
- US-A1- 2015 073 252
- GRIMNES S ED - ZARKOGIANNI KONSTANTIA ET AL: "IMPEDANCE MEASUREMENT OF INDIVIDUAL SKIN SURFACE ELECTRODES", MEDICAL AND BIOLOGICAL ENGINEERING AND COMPUT, SPRINGER, HEILDELBERG, DE, vol. 21, no. 6, 1 November 1983 (1983-11-01), pages 750-755, XP008076782, ISSN: 0140-0118, DOI: 10.1007/BF02464038
- Dorothee Grieshaber ET AL: "Electrochemical Biosensors -Sensor Principles and Architectures", Sensors, 1 January 2008 (2008-01-01), pages 1400-1458, XP055446655, Retrieved from the Internet: URL:https://www.ncbi.nlm.nih.gov/pmc/artic les/PMC3663003/pdf/sensors-08-01400.pdf [retrieved on 2008-01-01]
- KEVIN R. AROOM ET AL: "Bioimpedance Analysis: A Guide to Simple Design and Implementation", JOURNAL OF SURGICAL RESEARCH., vol. 153, no. 1, 1 May 2009 (2009-05-01), pages 23-30, XP055447096, US ISSN: 0022-4804, DOI: 10.1016/j.jss.2008.04.019
- Jose Carlos Conchell: "Bio-impedance circuit design for body worn systems", , 7 August 2014 (2014-08-07), pages 1-10, XP055447098, England DOI: 10.1016/j.medengphy.2006.02.005 Retrieved from the Internet: URL:http://www.analog.com/media/en/analog- dialogue/volume-48/number-4/articles/bio_i mp.pdf [retrieved on 2018-02-01]

## Description

### Technical Field

The present disclosure relates generally to a biosensor, and more particularly, to an electrochemical biosensor.

### Background Art

A biosensor is an analytical sensor that determines the concentration or presence of a biological analyte, such as glucose, cholesterol, lactate, creatinine, protein, hydrogen peroxide, alcohol, amino acids, glutamic-pyruvic transaminase (GPT), and glutamic-oxaloacetic transaminase (GOT). An electrochemical biosensor detects the flow or presence of electrons generated by electrochemical oxidation or reduction of the biological analyte.

When a biological analyte of a biosensor is included in a subject, the biological analyte may be harvested, such as through blood collection. However, in the conventional art, the subject suffers from repeated harvestings of the biological analyte. In addition, when the biological analyte is harvested, the state of the biological analyte varies, resulting in inaccurate calculation.

US 2011/077490 A1 relates to a transcutaneous analyte sensor.

### Technical Problem

Accordingly, there is a need in the art for a biosensor that eliminates the repeated harvestings of the biological analyte and more accurately calculates the biological analyte.

### Solution to Problem

The present disclosure has been made to address the above-mentioned problems and disadvantages, and to provide at least the advantages described below. The scope of the present invention is defined by the scope of appended independent entity claim 1 and independent activity claim 10. Preferred embodiments are defined by dependent claims 2-9, 11,12. Embodiments which do not fall under the scope of the appended claims are examples which are useful to understand the invention, but do not form part of the present invention.

An aspect of the present disclosure is to
provide a biosensor including a biosensor electrode structure that is capable of directly detecting a target material from a subject.

According to an aspect of the present disclosure, a biosensor electrode structure includes a working electrode that penetrates a subject, the working electrode including an enzyme that changes a first electrical response corresponding to a first electrical stimulation applied to the subject to a second electrical response in the subject, different than the first electrical response, and first and second impedance electrodes that are spaced apart from each other, contact the subject, and receive the first electrical response and the second electrical response from the subject.

According to another aspect of the present disclosure, a biosensor includes a working electrode that penetrates a subject and includes an enzyme that causes a target material to react, an impedance electrode part including a plurality of impedance electrodes that contact the subject and are spaced apart from each other, a first stimulator that provides a first electrical stimulation to the subject through the impedance electrode part, a second stimulator that provides a second electrical stimulation for activating the enzyme through the working electrode; and a first detector that detects an electrical response corresponding to at least one of the first and second electrical stimulations from the subject through the impedance electrode part.

According to another aspect of the present disclosure, a method of operating a biosensor having a working electrode that penetrates a subject and an enzyme for causing a target material to react, and a plurality of impedance electrodes that contact the subject and are spaced apart from one another, includes providing a first electrical stimulation to the subject through the plurality of impedance electrodes, detecting a first electrical response corresponding to the first electrical stimulation from the subject through the plurality of impedance electrodes, providing a second electrical stimulation to the subject through the working electrode, and detecting a second electrical response corresponding to the first electrical stimulation and the second electrical stimulation from the subject through the plurality of impedance electrodes.

### Brief Description of Drawings

The above and/or other aspects, features and advantages of the present disclosure will become apparent and more readily appreciated from the following description of the embodiments, taken in conjunction with the accompanying drawings in which:
FIG. 1 is a block diagram of a biosensor for detecting a target substance, according to an embodiment of the present disclosure;
FIG. 2a is a plan view of an electrode structure applicable to the biosensor of FIG. 1, according to an embodiment of the present disclosure;
FIG. 2b is a cross-sectional view of the electrode structure of FIG. 2a;
FIG. 3 is a plan view of a biosensor electrode structure having no separate reference electrode, according to an embodiment of the present disclosure;
FIG. 4 is a plan view of a pad-type biosensor electrode structure according to an embodiment of the present disclosure;
FIGS. 5a, 5b, 5c and 5d illustrate a method by which an enzyme is bonded to a needle electrode, according to an embodiment of the present disclosure;
FIG. 6a illustrates when a first electrical stimulation is not applied to first and second impedance electrodes, according to an embodiment of the present disclosure;
FIG. 6b illustrates when a first electrical stimulation is applied to first and second impedance electrodes, according to an embodiment of the present disclosure;
FIG. 6c illustrates when a first electrical stimulation is applied to first and second impedance electrodes and a second electrical stimulation is applied to a working electrode, according to an embodiment of the present disclosure;
FIG. 7 illustrates a biosensor electrode structure according to another embodiment of the present disclosure;
FIG. 8 illustrates an enzyme electrode according to another embodiment of the present disclosure;
FIG. 9 is a cross-sectional view of a biosensor electrode structure according to another embodiment of the present disclosure;
FIGS. 10, 11, 12 and 13 illustrate when an enzyme electrode is disposed at an impedance electrode, according to embodiments of the present disclosure;
FIG. 14 illustrates when needle electrodes are arranged at an impedance electrode, according to another embodiment of the present disclosure;
FIG. 15 illustrates a method of receiving a plurality of electrical responses in different regions of a subject, according to an embodiment of the present disclosure;
FIG. 16 illustrates a method of providing a plurality of electrical stimulations to different regions of a subject, according to another embodiment of the present disclosure;
FIG. 17 illustrates an electrode structure in which distances between impedance electrodes are different from one another, according to an embodiment of the present disclosure;
FIG. 18 illustrates a method of providing an electrical stimulation and detecting an electrical response in a biosensor, according to an embodiment of the present disclosure;
FIG. 19 illustrates a method of acquiring information about a target material in a biosensor, according to an embodiment of the present disclosure;
FIGS. 20 illustrates a method of operating a biosensor, according to another embodiment of the present disclosure;
FIG. 21a and 21b are reference diagrams for describing a method of removing a foreign material bonded to a biosensor electrode structure;
FIG. 22 is a block diagram of a biosensor having a current calculation function, according to an embodiment of the present disclosure;
FIG. 23 illustrates an example of a third electrical response with reference to time;
FIGS. 24a and 24b illustrate examples of first and second electrical responses with reference to time;
FIG. 25 is a plan view of an electrode structure applicable to a hybrid-type biosensor of FIG. 22;
FIG. 26a is a plan view of an electrode structure applicable to the hybrid-type biosensor of FIG. 22, according to another embodiment of the present disclosure;
FIG. 26b is a cross-sectional view of the electrode structure of FIG. 26a;
FIG. 27a is a plan view of an electrode structure applicable to the hybrid-type biosensor of FIG. 22, according to another embodiment of the present disclosure; and
FIG. 27b is a cross-sectional view of the electrode structure of FIG. 27a.

### Mode for the disclosure

Embodiments of the present disclosure will now be described in reference to the accompanying drawings, wherein like reference numerals refer to like elements throughout. In this regard, the present embodiments may have different forms and should not be construed as being limited to the descriptions set forth herein.

Rather, these embodiments are provided so that this disclosure will be thorough and complete, and will fully convey the concept to those of ordinary skill in the art. A description of well known functions and/or configurations will be omitted for the sake of clarity and conciseness.

The sizes of elements in the drawings may be exaggerated for convenience of explanation. In other words, since the sizes and thicknesses of components in the drawings are arbitrarily illustrated for convenience of explanation, the following embodiments are not limited thereto.

It will be understood that although terms such as "first" or "second" may be used herein to describe various components, these components should not be limited by these terms. Instead, these components are only used to distinguish one component from another. Expressions such as "at least one of" when preceding a list of elements, modify the entire list of elements and do not modify the individual elements of the list.

The terminology herein is used to describe particular embodiments only and is not intended to limit the scope of the inventive concept. As used herein, the singular forms "a", "an", and "the" are intended to include the plural forms as well, unless the context clearly indicates otherwise. It will be understood that terms such as "comprise", "include", and "have", when used herein, specify the presence of stated features, integers, steps, operations, elements, components, or combinations thereof, but do not preclude the presence or addition of one or more other features, integers, steps, operations, elements, components, or combinations thereof.

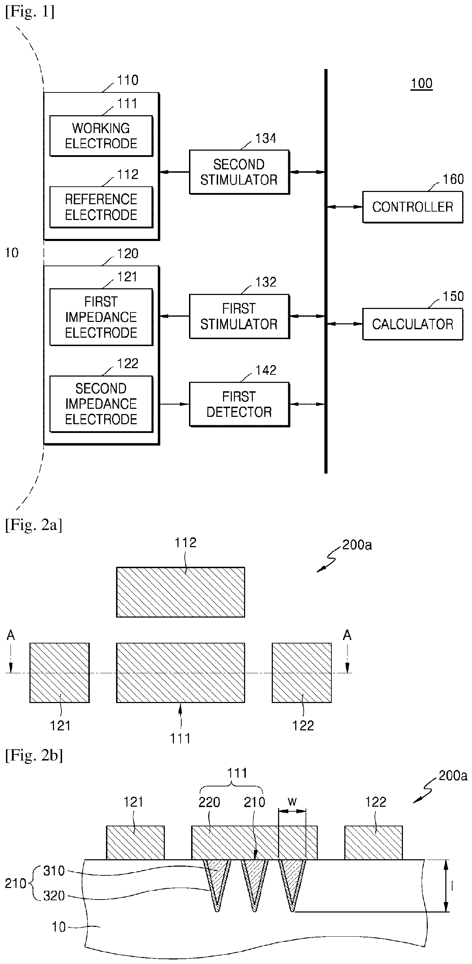

FIG. 1 is a block diagram illustrating a biosensor 100 that detects a target material, according to an embodiment of the present disclosure. Referring to FIG. 1, the biosensor 100 includes a working electrode part 110 and an impedance electrode part 120. The working electrode part 110 is a hardware element that penetrates a subject 10 and includes an enzyme that changes an electrical response corresponding to an electrical stimulation applied to the subject 10. The impedance electrode part 120 is a hardware element that contacts the subject 10, provides the electrical stimulation to the subject 10, and receives the electrical response and a changed electrical response.

Hereinafter, the electrical stimulation applied to the subject 10 through impedance electrode part 120 will be referred to as a first electrical stimulation, and an electrical stimulation for causing the enzyme of the working electrode 111 to work will be referred to as a second electrical stimulation. An electrical response corresponding to the first electrical stimulation will be referred to as a first electrical response, and an electrical response changed by the enzyme, that is, an electrical response corresponding to the first electrical stimulation and the second electrical stimulation, will be referred to as a second electrical response. Hereinafter, when an "electrical response" is simply referred to, the electrical response includes at least one of the first electrical response and the second electrical response.

The biosensor 100 further includes a first stimulator 132, a second stimulator 134, a first detector 142, and a calculator 150. The first stimulator 132 provides a first electrical stimulation to the subject 10 through the impedance electrode part 120. The second stimulator 134 provides a second electrical stimulation for activating the enzyme of the working electrode part 110. The first detector 142 detects the first electrical response and the second electrical response from the subject 10 through the impedance electrode part 120. The calculator 150 calculates a bioimpedance of the subject 10 by using the first electrical response and the second electrical response.

The subject 10 is a target of which a bioimpedance is to be calculated, and may be a human or a part of the human, and an animal or a part of the animal, a user or medical professional. An electrical response in the subject 10 is generated corresponding to an electrical stimulation. The user may also be a border concept other than the subject 10.

The first electrical stimulation may be a voltage and the first and second electrical responses may be a current. Thus, the voltage may be an alternating current (AC) voltage or an alternating current/direct current (AC/DC) voltage, but the present disclosure is not limited thereto, and the first electrical stimulation may be a current and the first and second electrical responses may be a voltage. Thus, the current may be AC or AC/DC.

The working electrode part 110 includes at least one working electrode 111 that penetrates the subject 10, and a reference electrode 112 serving as a reference for the potential of the working electrode 111. The working electrode 111 includes one or more enzyme electrodes, as shown in 210 of FIG. 2b, which has a needle shape with one sharp end so as penetrate the subject 10, and in which an enzyme is disposed on at least a partial surface thereof. The enzyme changes an electrical response corresponding to an electrical stimulation in the subject 10. A type of the enzyme varies depending on a type of a target material to be detected by the biosensor 100. The enzyme and the response of the target material will be described below.

The impedance electrode part 120 includes a plurality of impedance electrodes spaced apart from one another on a skin of the subject 10. The impedance electrodes may be classified into a complex-type electrode and a single-type electrode. When a first electrical electrode is applied to the subject 10 through one impedance electrode, the complex-type electrode receives an electrical response from the subject 10. When a first electrical electrode is applied to the subject 10 through one impedance electrode or through another impedance electrode.

When the impedance electrode is the complex-type electrode, the impedance electrode part 120 includes at least two impedance electrodes. For example, the impedance electrode part 120 includes first and second impedance electrodes 121 and 122 spaced apart from each other. A first electrical stimulation is applied to the subject 10 through the first and second impedance electrodes 121 and 122, and an electrical response is received from the subject 10 through the first and second impedance electrodes 121 and 122.

When a bioimpedance is calculated by using the complex-type electrode, the number of impedance electrodes is reduced, which simplifies the configuration of the biosensor 100. However, a contact impedance may occur due to a contact between the impedance electrode and the subject 10 when calculating the bioimpedance, and the bioimpedance may be affected according to a frequency of the first electrical stimulation. Therefore, a substantial load may be imposed on correction of the bioimpedance.

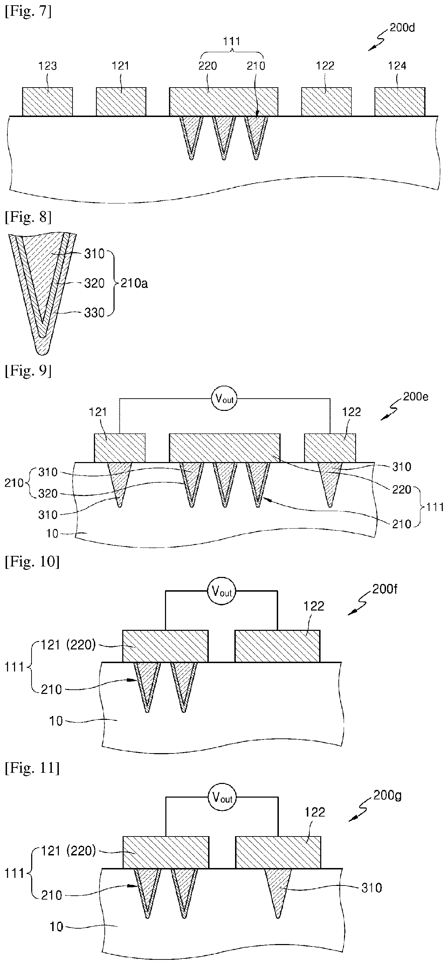

When the impedance electrode is the single-type electrode, the impedance electrode part 120 includes at least four impedance electrodes, such as first to fourth impedance electrodes 121, 122, 123, and 124 illustrated in FIG. 7, spaced apart from one another. For example, a first electrical stimulation is applied to the subject 10 through the first and second impedance electrodes 121 and 122, and an electrical response is received from the subject 10 through the third and fourth impedance electrodes 123 and 124.

Since the single-type impedance electrode indirectly calculates the bioimpedance, the contact impedance between the impedance electrode and the subject 10 is reduced. For example, when an input impedance of a voltage source that applies a voltage to the impedance electrode and an output impedance of an ammeter that detects a current of the impedance electrode are significantly greater than the contact impedance, influence of the impedance of the impedance electrode and the contact impedance may be minimized.

The impedance electrode may be a non-invasive electrode that contacts only the surface of the subject 10 and does not penetrate the subject 10, but the present disclosure is not limited thereto, and the impedance electrode may be an invasive electrode that penetrates the subject 10. For example, the non-invasive electrode has a plate shape so as to easily contact the skin of the subject 10, and the invasive electrode has a needle shape so as to easily penetrate the subject 10.

The first stimulator 132 provides the first electrical stimulation to the subject 10 through the impedance electrode part 120 and may be an AC voltage or a combination of an AC voltage and a DC voltage, but the present disclosure is not limited thereto, and the first electrical stimulation may be AC or AC/DC.

When the first stimulator 132 provides an AC voltage or an AC current as the first electrical stimulation, an operating frequency of the AC voltage or the AC current may have a sweep form or a single form. As the operating frequency is increased, it is possible to sense a deeper portion in the skin of the subject 10.

The operating frequency of the first electrical stimulation varies depending on a target material. For example, the operating frequency of the first electrical stimulation may be in the range of about 0 hertz (Hz) to about 1 gigahertz (GHz). When the first electrical stimulation is a voltage, the first stimulator 132 may be implemented by a voltage source. When the first electrical stimulation is a current, the first stimulator 132 may be implemented by a current source.

The second stimulator 134 provides the working electrode 111 with the second electrical stimulation for activating the enzyme. In order for the enzyme to react with a target material, a certain electrical stimulation is necessary, and may be a DC voltage or a DC current. The second electrical stimulation varies depending on the enzyme. For example, when the enzyme is glucose oxidase, the second electrical stimulation may be in the range of about 0.3 volts (V)to about 0.7 V, as compared to the reference electrode 112. When the enzyme is lactate oxidase, the second electrical stimulation may be about 0.6 V, as compared to the reference electrode 112.

The first detector 142 detects an electrical response from the subject 10 through the impedance electrode part 120. When the first electrical stimulation is a voltage, the first detector 142 is an ammeter that detects a current. When the first electrical stimulation is a current, the first detector 142 is a voltmeter that detects a voltage.

The calculator 150 calculates the bioimpedance of the subject 10 by using the first electrical stimulation, the first electrical response, and the second electrical response. Since each of all components of the subject 10 has an inherent resistance and an inherent permittivity, the bioimpedance varies depending on the components of the subject 10. Therefore, the biosensor 100 detects the presence or absence of a target material of the components of the subject 10, whether a concentration of the target material changes, or a concentration value of the target material, by using an impedance method.

Since the subject 10 includes various components, the calculated bioimpedance is a result of the combination of the various components included in the subject 10, making it difficult to analyze the target material included in the subject 10 by simply using the bioimpedance.

Thus, the biosensor 100 according to the present embodiment includes an enzyme electrode 210 that penetrates the subject 10 and includes an enzyme that causes the target material to chemically react in the subject 10, thus changing an electrical response in the subject 10. That is, the change in the electrical response is caused by the target material.

Specifically, the calculator 150 calculates a first bioimpedance by using the first electrical stimulation and the first electrical response and calculates a second bioimpedance by using the first electrical stimulation and the second electrical response. For example, when the first electrical stimulation is a voltage and the first and second electrical responses are a current, the calculator 150 calculates the bioimpedance by using a complex ratio of the voltage to the current or a real part of the complex ratio.

The controller 160 controls overall operations of the biosensor 100 and acquires information about the target material by using the bioimpedance calculated by the calculator 150. For example, the controller 160 controls the first stimulator 132 to provide the first electrical stimulation to the subject 10 and control the second stimulator 134 to provide the second electrical stimulation to the enzyme. In addition, the controller 160 controls the first detector 142 to detect the first electrical response in when the enzyme is deactivated, and controls the first detector 142 to detect the second electrical response in when the enzyme is activated. The controller 160 controls the calculator 150 to calculate the bioimpedance.

The controller 160 acquires information about the target material by using a change amount of the first bioimpedance and the second bioimpedance calculated by the calculator 150. For example, when the change amount of the first bioimpedance and the second bioimpedance is less than a reference value, the controller 160 determines that the target material is absent in the subject 10. When the change amount of the first bioimpedance and the second bioimpedance is equal to or greater than the reference value, the controller 160 determines that the target material is present in the subject 10.

In addition, the controller 160 determines whether the target material is changed, based on a change in the change amount with reference to time. For example, when the change in the change amount decreases with reference to time, the controller 160 determines that the concentration of the target material in the subject 10 decreases. When the change in the change amount increases with reference to time, the controller 160 determines that the concentration of the target material in the subject 10 increases.

The controller 160 performs quantitative analysis for the target material, in which the controller 160 refers to a lookup table in which a relationship between the change in the bioimpedance and the concentration of the target material is defined. The lookup table may be prestored in the biosensor 100. The controller 160 uses the lookup table stored in an external device. In this case, the biosensor 100 further includes a communicator that is capable of communicating with the external device.

The analysis for the target material may be performed by the biosensor 100, or the biosensor 100 may calculate only the bioimpedance and transmit a result of the calculation to the external device, such as a mobile phone which analyzes the target material. In this case, the biosensor 100 does not include the controller 160, and instead, includes a communicator that is capable of communicating with the external device. In addition, the calculator 150 that is capable of calculating the bioimpedance may be included in the external device. The external device operates as a master device that controls the biosensor 100, and the biosensor 100 operates as a slave device under the control of the external device.

FIG. 2a is a plan view of an electrode structure 200a applicable to the biosensor 100 of FIG. 1, according to an embodiment of the present disclosure and FIG. 2b is a cross-sectional view of the electrode structure 200a of FIG. 2a. Hereinafter, the electrode structure 200a applicable to the biosensor 100 is referred to as a biosensor electrode structure 200a.

Referring to FIGS. 2a and 2b, the biosensor electrode structure 200a includes first and second impedance electrodes 121 and 122 spaced apart from each other, and a working electrode 111 between the first and second impedance electrodes 121 and 122. The first and second impedance electrodes 121 and 122 are symmetrical to the working electrode 111, or a shape of the first impedance electrode 121 is symmetrical to a shape of the second impedance electrode 122 with respect to the working electrode 111.

The first and second impedance electrodes 121 and 122 are attachable to or detachable from the skin surface of the subject 10. Each of the first and second impedance electrodes 121 and 122 has a plate shape of which a cross-section is relatively greater than a length 1 thereof. Therefore, the first and second impedance electrodes 121 and 122 are easily attachable to or detachable from the skin surface of the subject 10. The cross-sections of the first and second impedance electrodes 121 and 122 are illustrated as being rectangular, but are not limited thereto. The cross-sections of the first and second impedance electrodes 121 and 122 may have various shapes, such as a circle, an oval, or a polygon.

The first and second impedance electrodes 121 and 122 includes a conductive material, such as a metal or a conductive metal oxide. For example, each of the first and second impedance electrodes 121 and 122 includes a metal, such as a metal incuding titanium (Ti), plutonium (Pt), rhodium (Ru), gold (Au), silver (Ag), molybdenum (Mo), aluminum (Al), tungsten (W), or copper (Cu), or a metal oxide, such as indium tin oxide (ITO), aluminum zinc oxide (AZO), indium zinc oxide (IZO), tin oxide (SnO₂), indium oxide (In₂O₃)_{'} or silver chloride (AgCl).

Alternatively, the first and second impedance electrodes 121 and 122 are formed by coating a conductive material on a certain material. For example, the first and second impedance electrodes 121 and 122 are formed by coating a conductive material on a polymer material.

The biosensor 100 according to the present embodiment may be used to detect the target material in the skin of the subject 10. The skin may be divided into an epidermis, a dermis, and a subcutaneous layer when seen from the outside. The epidermis may have a waterproof function and serve as a protective barrier to infection. The epidermis may be supplied with nourishment by diffusion of nourishment from the dermis. The dermis may be a space for appendages of the skin and protects the subject 10 against stress and strain through buffering. The dermis includes interstitial fluid (ISF) and capillaries. The subcutaneous layer may have large amounts of adipose tissue and store nourishment. Therefore, a large amount of target material used to determine health states may be present in the dermis and the subcutaneous layer.

Therefore, the biosensor 100 according to the present embodiment may be used to detect a target material from the skin of the subject 10, and in particular, a target material included in the dermis and the subcutaneous layer. Since the dermis and the subcutaneous layer are placed beneath the epidermis, the biosensor 100 according to the present embodiment includes the working electrode 111 that penetrates the subject 10, for example, up to the dermis and the subcutaneous layer. In addition, since the interstitial fluid and the target material are present in the epidermis, the biosensor according to the present embodiment penetrates the epidermis of the subject 10 and detects the bioimpedance.

The working electrode 111 is spaced apart from the first and second impedance electrodes 121 and 122 or is disposed between the first and second impedance electrodes 121 and 122. The working electrode 111 includes one or more enzyme electrodes 210, which has one sharp end so as to penetrate the subject 10, and in which an enzyme is disposed on at least a partial surface thereof. Since the working electrode 111 penetrates the subject 10, the working electrode 111 will be referred to an invasive-type working electrode.

The length 1 of the enzyme electrode 210 varies depending on an invasion depth with respect to the subject 10. For example, the enzyme electrode 210 penetrates the subject 10 up to the dermis through the epidermis. Since a human rarely feels pain up to the dermis of the human body, the enzyme electrode 210 may have a length 1 so that the enzyme electrode 210 penetrates the dermis. When attempting to detect the target material in the dermis, the length 1 of the enzyme electrode may be in the range of about 70 µm to about 1,400 *µ*m.

A depth range in which the dermis of the subject 10 is distributed varies depending on the portions of the subject 10. For example, in the abdomen of the human body, the thickness of the epidermis may be about 79.4 *µ*m ± 33.9 *µ*m, and the thickness of the dermis may be about 1,248.4 *µ*m ± 262.5 *µ*m. In addition, in the rear of the arm of the human body, the thickness of the epidermis may be about 83.5 *µ*m ± 36.2 *µ*m, and the thickness of the dermis may be about 1,030.4 *µ*m ± 327.8 *µ*m. Therefore, the enzyme electrode 210 penetrates the subject 10 from the skin surface of the subject 10 up to about a depth of about 70 *µ*m to about 1,300 *µ*m.

An example in which the enzyme electrode 210 penetrates the subject 10 up to the dermis has been described above, but the present embodiment is not limited thereto. For example, the enzyme electrode 210 penetrates the subject 10 up to the epidermis. In this case, the length 1 of the enzyme electrode 210 may be about 70 *µ*m. For example, the enzyme electrode 210 penetrates the subcutaneous layer. In this case, the length 1 of the enzyme electrode 210 may be greater than about 1,400 *µ*m.

The maximum width w of the enzyme electrode 210 may be less than the length 1. The maximum width w of the enzyme electrode 210 may be so small as to cause less pain when the enzyme electrode 210 penetrates the subject 10. For example, the maximum width w of the enzyme electrode 210 may be about dozens to hundreds of *µ*m (about 500 *µ*m). The maximum width w of the enzyme electrode 210 may be in the range of about 40 *µ*m to about 60 *µ*m. The above numerical values are only an example and the present embodiment is not limited thereto. In addition, the maximum width w of the enzyme electrode 210 varies depending on a position of a target material to be detected or a type of the subject 10, for example.

The enzyme electrode 210 will be described in detail. The enzyme electrode 210 includes a needle electrode 310 having a needle shape of which the length 1 is longer than the cross-section thereof, and a reagent layer 320 in which a reagent is provided on the surface of the needle electrode 310. The reagent layer 320 includes an enzyme.

One end of the needle electrode 310 may be sharp. Therefore, the one end of the enzyme electrode 210 may easily penetrate the subject 10. The needle electrode 310 may have a tapered shape, of which one end is sharp. For example, as illustrated in FIG. 2b, the needle electrode 310 may have a shape of which the width becomes gradually narrow toward one end from the other end, but the present disclosure is not limited thereto, and the width of the needle electrode 310 may be uniform in a partial portion thereof and may gradually decrease toward one end in the remaining portion.

The needle electrode 310 receives the second electrical stimulation for activating an enzyme from the second stimulator 134. The second electrical stimulation may be a voltage or a current. For example, the second electrical stimulation may be a DC voltage or a DC current. The magnitude of the second electrical stimulation varies depending on a type of an enzyme.

The width and length of the needle electrode 310 may be determined in consideration of, for example, an amount of enzyme, a region of the subject 10 from which a target material is to be detected, or pain that the subject 10 feels when the needle electrode 310 penetrates the subject 10. For example, when the width of the needle electrode 310 is large, a large amount of enzyme is provided in the needle electrode 310. In this case, when the needle electrode 310 penetrates the subject 10, the subject 10 may feel pain. When the length 1 of the needle electrode 30 is short, the enzyme may not penetrate a position in which the target material is present.

The cross-section of the needle electrode 310 may have a polygon, such as triangle or rectangle, a circle, or an oval, but is not limited thereto. Generally, the size of the needle electrode 310 determines the size of the enzyme electrode 210.

The needle electrode 310 includes a material having high electrical conductivity-includes, such as a metal incuding titanium (Ti), plutonium (Pt), rhodium (Ru), gold (Au), silver (Ag), molybdenum (Mo), aluminum (Al), tungsten (W), or copper (Cu), or is formed by coating the metal on a certain material. The needle electrode 310 may have conductivity equal to or greater than those of the first and second impedance electrodes 121 and 122.

The reagent layer 320 is disposed in at least a partial portion of the needle electrode 310. The reagent layer 320 includes an enzyme for causing the target material to react. The target material may be distributed in, for example, the interstitial fluid in the subject 10. When the second electrical stimulation that is uniform is applied to the needle electrode 310, the enzyme of the reagent layer 320 is activated. The second electrical stimulation is provided by a voltage between the working electrode 111 and the reference electrode 112.

The activated enzyme causes the target material floating in the interstitial fluid to react, thus generating a reactant. The reactant changes an electrical response in the subject 10. For example, the reactant changes the electrolytic component of the interstitial fluid, and a change in the electrolytic component changes a current amount of the interstitial fluid, that is, an electrical response.

The changed electrical response may depend on the amount of the target material. For example, as the amount of the target material increases, the electrical response may greatly change. Therefore, the biosensor 100 acquires information about the target material based on the change amount of the bioimpedance.

The enzyme of the reagent layer 320 varies depending on the type of the target material. When the target material is glucose, the enzyme may be at least one of glucose oxidase and glucose dehydrogenation enzyme. When the target material is cholesterol, the enzyme may be cholesterol oxidase or cholesterol esterifying enzyme. The reagent layer 320 further includes coenzyme. The coenzyme may help the enzyme react with the target material. The coenzyme may be, for example, flavin adenine dinucleotide (FAD) or nicotinamide adenine dinucleotide (NAD).

The working electrode 111 further includes a support electrode 220 that supports the at least one enzyme electrode 210. The support electrode 220 is attachable to or detachable from the skin surface of the subject 10. The support electrode 220 has a plate shape of which the cross-section is relatively longer than the length thereof. Therefore, the support electrode 220 is easily attachable to or detachable from the skin surface of the subject 10. The cross-section of the support electrode 220 is illustrated as being rectangular, but the present disclosure is not limited thereto, and the cross-section of the support electrode 220 may have various shapes, such as a circle, an oval, and a polygon.

The support electrode 220 includes a conductive material, such as a metal or a conductive metal oxide. For example, the support electrodes 220 may be a metal such as Ti, Pt, Ru, Au, Ag, Mo, Al, W, or Cu, or a metal oxide such as indium tin oxide (ITO), aluminum-doped zinc oxide (AZO), indium-zinc oxide (IZO), tin oxide (SnO₂), or indium (III) oxide (In₂O₃). The support electrode 220 is formed by coating a conductive material on a certain material such as a polymer.

The surface of the support electrode 220 that contacts the skin surface of the subject 10 may contact the other end of the enzyme electrode 210. The enzyme electrode 210 is disposed to be perpendicular to the support electrode 220, but the present disclosure is not limited thereto, and the enzyme electrode 210 may be inclined with respect to the support electrode 220 at a certain angle.

At least one enzyme electrode 210 is disposed at a single support electrode 220. When a plurality of enzyme electrodes 210 is disposed at the support electrode 220, the plurality of enzyme electrodes 210 may be arranged in a one-dimensional manner or a two-dimensional manner.

The plurality of enzyme electrodes 210 has substantially the same size and shape, but the present disclosure is not limited thereto, and at least two of the plurality of enzyme electrodes 210 may have different sizes and shapes. In this case, the enzyme provided in the enzyme electrode 210 is provided in a wider range in the subject 10. In addition, the plurality of the enzyme electrodes 210 is arranged at uniform intervals or at non-uniform intervals. The arrangement, sizes and shapes of the enzyme electrodes 210 are determined in various manners according to the type of the target material and the state of the subject 10, for example.

Since the support electrode 220 serves to support the enzyme electrode 210 and provide the second electrical stimulation to the enzyme electrode 210, the support electrode 220 may not be an essential component. That is, the working electrode 111 may include only the enzyme electrode 210, and another electrode serves as the support electrode 220.

The reference electrode 112 is spaced apart from the working electrode 111 and from the first and second impedance electrodes 121 and 122, but the present disclosure is not limited thereto, and one of the first and second impedance electrodes 121 and 122 serves as the reference electrode 112.

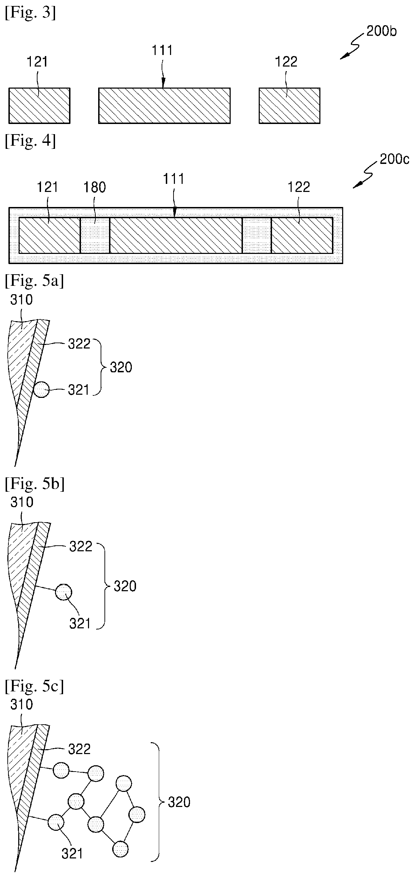

FIG. 3 is a plan view of a biosensor electrode structure 200b having no separate reference electrode, according to an embodiment of the present disclosure. Referring to FIG. 3, the electrode structure 200b does not include the reference electrode 112, and at least one of the first and second impedance electrodes 121 and 122 serves as the reference electrode 112.

FIG. 4 is a plan view of a pad-type biosensor electrode structure 200c according to an embodiment of the present disclosure. Referring to FIG. 4, the biosensor electrode structure 200c is connected by an insulating material 180. Therefore, electrodes included in the biosensor electrode structure 200c are spaced apart from one another at uniform intervals. Since the biosensor electrode structure 200c is formed to have a single pad type, the biosensor electrode structure 200c is easily attachable to or detachable from the subject 10.

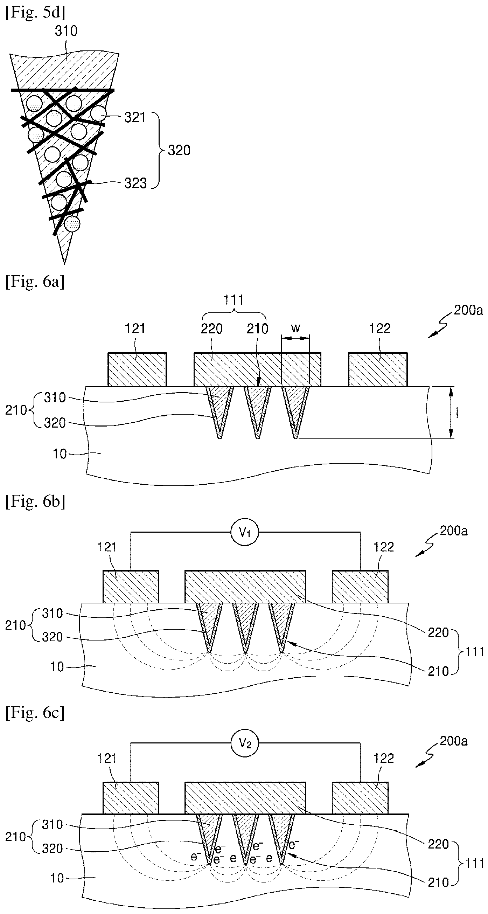

FIGS. 5a, 5b, 5c and 5d illustrate a method by which an enzyme 321 is bonded to a needle electrode 310, according to an embodiment of the present disclosure. Referring to FIGS. 5a to 5c, the enzyme 321 is bonded to the needle electrode 310 by a resin 322 that is coated on the surface of the needle electrode 310. As illustrated in FIG. 5a, the enzyme 321 is bonded to the resin 322 by being adsorbed onto the resin 322. As illustrated in FIG. 5a, the enzyme 321 is bonded to the resin 322 through a covalent bond, in which case a bonding strength may be higher than that in bonding by adsorption.

As illustrated in FIG. 5c, some of the enzyme 321 is bonded to the resin 322 through a covalent bond and the remaining portion of the enzyme 321 is bonded to another adjacent enzyme through a covalent bond. As described above, due to the crosslinking between the enzymes 321, more enzyme 321 is provided in the needle electrode 310.

As illustrated in FIG. 5d, the enzymes 321 are bonded by coating polymers 323 on the needle electrode 310 and encapsulating the enzyme 321 between the polymers 323. The bonding of the enzymes 321 by using the polymer 323 facilitates the manufacturing, as compared with the bonding of the enzymes 321 by using the covalent bond.

FIG. 6a illustrates when a first electrical stimulation, for example, a voltage, is not applied to first and second impedance electrodes 121 and 122, according to an embodiment of the present disclosure. As illustrated in FIG. 6a, since the first electrical stimulation is not applied to the first and second impedance electrodes 121 and 122, a first electrical response corresponding to the first electrical stimulation is not formed in the subject 10 between the first and second impedance electrodes 121 and 122.

FIG. 6b illustrates when a first electrical stimulation is applied to the first and second impedance electrodes 121 and 122, according to an embodiment of the present disclosure. The first electrical stimulation may be an AC voltage. The operating frequency may have a sweep form, but the present disclosure is not limited thereto. That is, when a first electrical stimulation is applied to the first and second impedance electrodes 121 and 122, the biosensor 100 and the subject 10 may form a closed circuit around the first and second impedance electrodes 121 and 122.

For example, when a voltage is applied to the first and second impedance electrodes 121 and 122, a current path is formed in the subject 10 around the first and second impedance electrodes 121 and 122. The current path varies according to the state of the subject 10. Since the subject 10 is a type of impedance, a value of a current flowing through the subject 19 varies according to components of materials in the subject 10, such as fat or moisture. The current path corresponds to the first electrical response V1. In FIG. 6b, since the first electrical stimulation is applied to the first and second impedance electrodes 121 and 122 and the second electrical stimulation is not applied to the working electrode 111, the enzyme is deactivated.

FIG. 6c illustrates when a first electrical stimulation is applied to first and second impedance electrodes 121 and 122 and a second electrical stimulation is applied to a working electrode 111, according to an embodiment of the present disclosure. Referring to FIG. 6c, the second electrical stimulation, such as a DC voltage for activating the enzyme, is applied to the working electrode 111. The magnitude of the voltage may be determined by setting the reference electrode 112 as a reference. When the second electrical stimulation is applied to the working electrode 111, the enzyme is activated to cause the target material to react, generating reactant. The reactant changes the electrolytic component of the interstitial fluid and a change in electrolytic component changes a current of the interstitial fluid from a first electrical response V1 to a second electrical response V2.

For example, glucose oxidase is provided in the working electrode 111, to which the second electrical stimulation is applied. In this case, the glucose oxidase is activated, causing a target material adjacent to the working electrode 111, i.e., glucose, to react with oxygen to generate reactant. Specifically, the glucose oxidase causes glucose to react with oxygen to generate gluconic acid and hydrogen peroxide (H₂O₂) as expressed in the following Formula (1). The hydrogen peroxide may be decomposed to generate electrons.

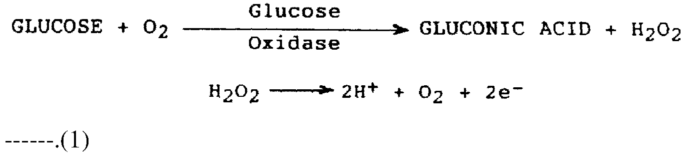

For example, lactate oxidase is provided in the working electrode 111. The second electrical stimulation, such as a DC voltage of about 0.6 V, is applied to the working electrode 111. In this case, the lactate oxidase is activated, causing a target material adjacent to the working electrode 111, i.e., lactate, to react with oxygen to generate reactant. Specifically, the lactate oxidase causes glucose with react with oxygen to generate pyruvate and hydrogen peroxide H₂O₂ as expressed in the following Formula (2). The hydrogen peroxide may generate electrons by being broken.

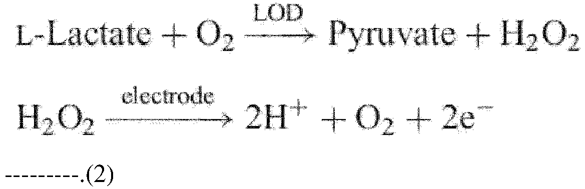

Since the first electrical stimulation is applied to the first and second impedance electrodes 121 and 122, a current path is formed in the subject 10 between the first and second impedance electrodes 121 and 122. The current path varies by the electrons generated by the enzyme. That is, since the electrolytic component of the interstitial fluid is changed by the electrons, the current value of the current path varies. For example, the second electrical response V2 detected when the enzyme is activated is greater than the first electrical response V1 when the enzyme is deactivated.

A change amount the first electrical response and the second electrical response is caused by the target material distributed in the skin of the subject 10. The magnitude of the change amount may be proportional to the amount of the target material. Therefore, it is possible to determine whether the target material is present, whether the target material is changed, or the concentration of the target material, based on the change amount of the first and second electrical responses.

It has been described with reference to FIG. 6c that the first electrical stimulation is applied to the first and the second impedance electrodes 121 and 122 in when the second electrical stimulation is applied to the working electrode 111, but the present disclosure is not limited thereto. That is, the second electrical stimulation may be applied to the working electrode 111 for a period of time and the first electrical stimulation may be applied to the first and second impedance electrodes 121 and 122 immediately after application of the second electrical stimulation is stopped. As described above interference of the first electrical stimulation and the first electrical stimulation may be reduced by alternately applying the second electrical stimulation and the first electrical stimulation.

FIG. 7 illustrates a biosensor electrode structure 200d according to another embodiment of the present disclosure. Referring to FIG. 7, the biosensor electrode structure 200d further includes third and fourth impedance electrodes 123 and 124 spaced apart from each other. The first and second impedance electrodes 121 and 122 and the working electrode 111 are disposed between the third and fourth impedance electrodes 123 and 124, which are disposed to be symmetrical to the working electrode 111 and have a shape symmetrical to the working electrode 111.

The third and fourth impedance electrodes 123 and 124 have substantially the same size and shape as the first and second impedance electrodes 121 and 122. Specifically, the third and fourth impedance electrodes 123 and 124 are attachable to or detachable from the surface of skin of the subject 10. Each of the third and fourth impedance electrodes 123 and 124 has a plate shape of which the cross-section is relatively greater than the length thereof. Therefore, the first and second impedance electrodes 121 and 122 are easily attachable to or detachable from the skin surface of the subject 10. The third and fourth impedance electrodes 123 and 124 include or are coated with a conductive material.

The first electrical stimulation is applied to the subject 10 through the third and fourth impedance electrodes 123 and 124. In this case, a current path is formed in the subject 10 between the third and fourth impedance electrodes 123 and 124. The first electrical response and the second electrical response are detected through the first and second impedance electrodes 121 and 122, respectively. As described above, the electrode to which the electrical stimulation is applied is separated from the electrode though which the electrical response is detected, thereby reducing a contact resistance between the electrode and the subject 10 and therefore, reducing noise.

The first electrical stimulation is applied to the subject 10 through the first and second impedance electrodes 121 and 122, and is applied to subject 10 through the third and fourth impedance electrodes 123 and 124. For convenience of description, it is assumed that an impedance electrode to be described below has a complex-type. However, the present embodiment is not limited thereto. The following description will focus on reception of an electrical response although the electrode to which the first electrical stimulation is different from the electrode that receives the electrical response.

FIG. 8 illustrates an enzyme electrode 210a according to another embodiment of the present disclosure. When an enzyme of the enzyme electrode 210a is exposed to the outside, a foreign material may be combined with the enzyme, or the enzyme may be damaged by external stimulation. As illustrated in FIG. 8, the enzyme electrode 210a further includes a protection layer 330 that covers the reagent layer 320. The protection layer 330 protects the enzyme until the enzyme penetrates the subject 10.

The protection layer 330 includes a material that is decomposable when penetrating the subject 10, such as a biodegradable polymer material decomposed in the subject 10. The biodegradable polymer material includes at least one selected from polylactic acid, poly(lactic-co-glycolic) acid, poly(caprolactone), polyhydroxyalkanoates, poly(propylene fumarate), polydioxanone, polyglycolide, polyanhydrides, polyacetals, poly(ortho esters), polycarbonates, polyurethanes, and polyphosphazenes. The aforementioned biodegradable polymer materials are only an example and the present disclosure is not limited thereto. When the enzyme electrode 210 penetrates the subject 10, the biodegradable polymer material is decomposed in the Interstitial fluid or blood of the subject 10, thereby exposing the enzyme to the subject 10.

Alternatively, the protection layer 330 may include a permeable material which is permeable to water or a target material, such as Nafion. The target material or water penetrates the protection layer 330 to react with the enzyme electrode 210, and the protection layer 300 prevents a foreign material larger than the target material from being adsorbed onto the enzyme electrode 210. Therefore, a reduction in detection sensitivity of the enzyme electrode 210 that is caused by adsorption is suppressed.

FIG. 9 illustrates a biosensor electrode structure 200e according to another embodiment of the present disclosure. Referring to FIG. 9, one or more needle electrodes 310, which penetrate the subject 10, are further disposed in at least one of the first and second impedance electrodes 121 and 122. The needle electrode 310 has one end that is sharp and another end that contacts the impedance electrode. The needle electrodes 310 are illustrated as being disposed at both the first and second impedance electrodes 121 and 122, but present disclosure is not limited thereto, and the needle electrode 310 may be further disposed at one of the first and second impedance electrodes 121 and 122. Since the needle electrodes 310 are disposed at the first and second impedance electrodes 121 and 122, an electrode that calculates an impedance will be referred to as an "invasive electrode".

The length and width of the needle electrode 310 correspond to the length and width of the working electrode 111. Specifically, the length of the needle electrode 310 varies depending on a depth up to which the needle electrode 310 penetrates the subject 10. For example, the needle electrode 310 penetrates the dermis of the subject 10. Since the subject 10 rarely feels pain in the dermis of a human body, the needle electrode 310 is not burdensome on the subject 10 even when the needle electrode 310 penetrates the dermis. When attempting to detect a target material from the dermis, the length of the needle electrode 310 may be in the range of about 70 microns (*µ*m) to about 1,400 *µ*m.

The width w of the needle electrode 310 is less than the length of the needle electrode 310, to an extent that pain is minimized when the needle electrode 310 penetrates the subject 10. For example, the maximum width of the needle electrode 310 may be in the range of about 40 *µ*m to about 60 *µ*m. The above numerical values are only an example and the present embodiment is not limited thereto. The cross-section of the needle electrode 310 may have a polygon, such as triangle or rectangle, a circle, or an oval, but present disclosure is not limited thereto.

The needle electrode 310 includes a material having high electrical conductivity, such as a metal including Ti, Pt, Ru, Au, Ag, Mo, Al, W, or Cu, or is formed by coating the metal on another material. The needle electrode 310 may have conductivity equal to or higher than the conductivity of the first and second impedance electrodes 121 and 122.

Since the needle electrode 310 is disposed at the impedance electrode, an electric field between the needle electrode 310 and the working electrode 111 may be further uniform than an electric field between the impedance electrode in which the needle electrode 310 is not disposed and the working electrode 111. Due to the uniform electric field, it may be possible to calculate a bioimpedance more precisely. In addition, the current path may not be formed up to the skin surface of the subject 10 and therefore, noise caused by dead skin cells is reduced.

FIGS. 10, 11, 12 and 13 illustrate when an enzyme electrode is disposed at an impedance electrode, according to embodiments of the present disclousre. Referring to FIG. 10, the enzyme electrode 210 is disposed at one of the first and second impedance electrodes 121 and 122. For example, the enzyme electrode 210 is disposed at the first impedance electrode 121, which serves as the support electrode 220. One end of the enzyme electrode 210 has a needle shape and the other end thereof contacts the first impedance electrode 121. Since the first impedance electrode 121 also provides the second electrical stimulation to the enzyme electrode, the electrode structure of the biosensor 100 is simplified. When the enzyme electrode 210 is disposed at the impedance electrode, the enzyme electrode 210 and the impedance electrode are collectively referred to as the working electrode 111.

The second stimulator 134 of the biosensor 100 may be connected to the first impedance electrode and the reference electrode 112, as illustrated in FIG. 1. Therefore, the second stimulator 134 provides the second electrical stimulation to the enzyme electrode 210 through the first impedance electrode 121. In addition, the first stimulator 132 is connected to the first and second impedance electrodes 121 and 122. Therefore, the first stimulator 132 provides the first electrical stimulation to the subject 10 through the first and second impedance electrodes 121 and 122.

The second impedance electrode 122 serves as the reference electrode 112. In this case, the first stimulator 132 and the second stimulator 134 are implemented as a single stimulator which provides the first electrical stimulation to the subject 10 through the first and second impedance electrodes 121 and 122 and provides the second electrical stimulation to the enzyme electrode 210. For example, the stimulator simultaneously provides the first and second first electrical stimulations by a combination of a DC voltage and an AC voltage or provides the first electrical stimulation by an AC voltage. In this case, the stimulator may be implemented by a voltage source, but the present disclosure is not limited thereto, and the stimulator may provide the first and second electrical stimulations in the form of current implemented by a current source.

Referring to FIG. 11, one or more needle electrodes are further disposed at the second impedance electrode 122. The needle electrode 310 has one end that is sharp, and the other end that contacts the second impedance electrode. The length and width of the needle electrode 310 correspond to the length and width of the enzyme electrode 210. In the biosensor electrode structure 200g illustrated in FIG. 11, the first and second stimulators 132 and 134 are separately included, or a single stimulator isincluded. Since the needle electrodes 310 are disposed at the second impedance electrode, an electric field is more uniformly formed in the subject 10.

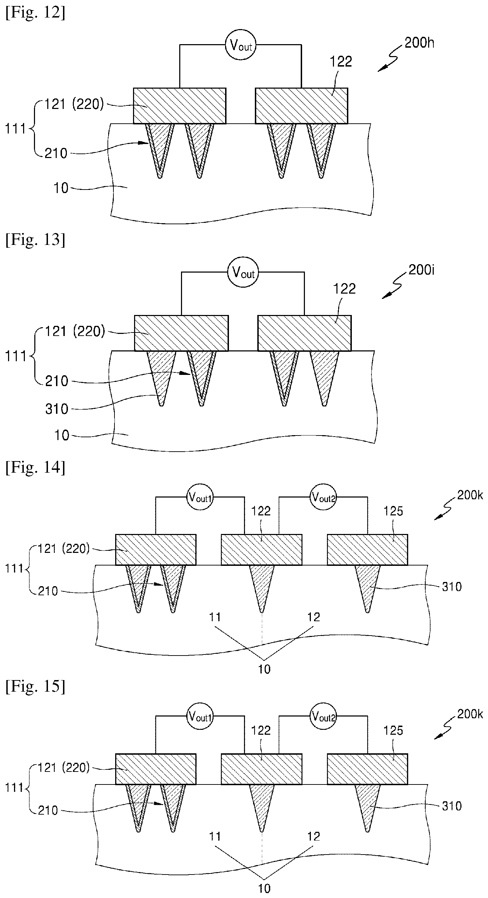

Alternatively, as illustrated in FIG. 12, a plurality of enzyme electrodes 210 is disposed at both the first and second impedance electrodes 121 and 122. Thus, a plurality of enzyme electrodes 210 is integrated, thereby causing a substantial amount of target material to react.

Alternatively, as illustrated in FIG. 13, the combination of the needle electrodes 310 and the enzyme electrodes 210 is alternately disposed in at least one of the first and second impedance electrodes 121 and 122. The needle electrodes 310 and the enzyme electrodes 210 are arranged in a one-dimensional manner or a two-dimensional manner, and symmetrically or alternately with respect to a central axis of the first and second impedance electrodes 121 and 122.

FIG. 14 illustrates when the needle electrode 310 is disposed at the impedance electrode, according to another embodiment of the present disclosure. As illustrated in FIG. 14, a plurality of needle electrodes 310 is disposed at the first and second impedance electrodes 121 and 122. The needle electrode 310 has one end that is sharp, and the other end that contacts the first and second impedance electrodes 121 and 122. When a plurality of needle electrodes 310 is disposed at a single impedance electrode, the needle electrodes 310 are arranged in a one-dimensional manner or a two-dimensional manner and are disposed at the first impedance electrode 121. The needle electrodes 310 disposed at the second impedance electrode 122 are arranged symmetrically with respect to a center between the first and second impedance electrodes 121 and 122.

As illustrated in FIG. 14, since the needle electrodes 310 penetrate the subject 10, noise caused by dead skin cells in the surface of the subject 10 is removed from the calculated bioimpedance. Therefore, the biosensor 100 calculates the bioimpedance at a specific point on the skin of the subject 10.

FIG. 15 illustrates a method of receiving a plurality of electrical responses to different regions of the subject, according to an embodiment of the present disclosure. Referring to FIG. 15, a biosensor electrode structure 200k further includes a fifth impedance electrode 125 spaced apart from the second impedance electrode 122. A distance between the second impedance electrode 122 and the fifth impedance electrode 125 is substantially equal to a distance between the first impedance electrode 121 and the second impedance electrode 122. The needle electrode 310 disposed at the fifth impedance electrode 125 has a length and a width corresponding to the length and width of the needle electrode 310 disposed at the second impedance electrode 122.

When a first region 11 has a material configuration similar to that of a second region 12 in the subject 10, the bioimpedance of the first region 11 is substantially equal to the bioimpedance of the second region 12 when the enzyme is deactivated. The bioimpedance varies due to changes in the environment, such as humidity or temperature.

The bioimpedance according to the change in environment is detected by using the second impedance electrode 122 and the fifth impedance electrode 125. The bioimpedance detected from the first impedance electrode 121 and the second impedance electrode 122 may be corrected by using the detected bioimpedance.

The needle electrodes 310 are illustrated as being disposed at the second and fifth impedance electrodes 122 and 125, respectively, but the present embodiment is not limited thereto, and the needle electrodes 310 may not be disposed at the respective second and fifth impedance electrodes 122 and 125.

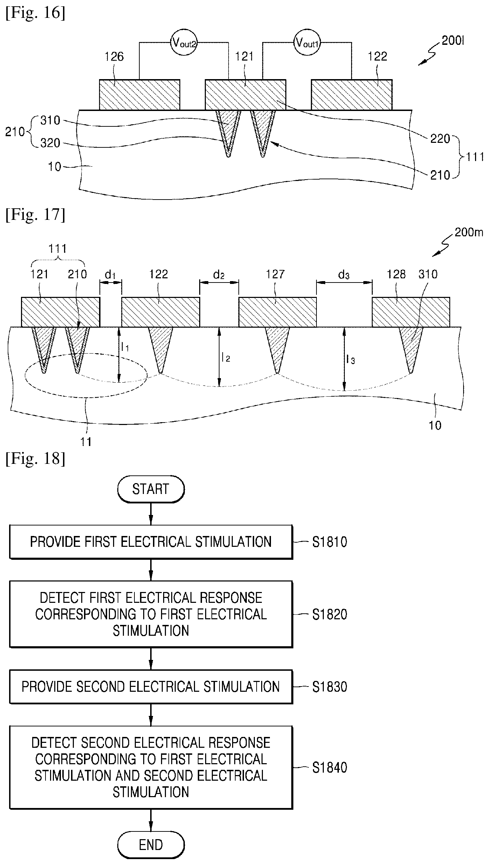

FIG. 16 illustrates a method of providing a plurality of electrical stimulations to different regions of the subject 10, according to another embodiment of the present disclosure. Referring to FIG. 16, a sixth impedance electrode 126 is arranged to be symmetrical to the second impedance electrode 122 with respect to the first impedance electrode 121. A distance between the first impedance electrode 121 and the sixth impedance electrode 126 is substantially equal to a distance between the first impedance electrode 121 and the second impedance electrode 122.

The biosensor 100 accurately determines information about a target material by calculating bioimpedances of a plurality of regions in the subject 10. For example, the biosensor 100 calculates a change amount of the bioimpedance by using the first and second impedance electrodes 121 and 122 (hereinafter, a "first change amount"). In addition, the biosensor 100 calculates a change amount of the bioimpedance by using the first and sixth impedance electrodes 121 and 126 (hereinafter, a "second change amount"). Noise is reduced by determining the average of the first change amount and the second change amount as a final change amount of the bioimpedance. The bioimpedances for two regions are illustrated as being calculated in the drawings, but the present disclosure is not limited thereto, and the bioimpedances for three or more regions may be calculated.

FIG. 17 illustrates an electrode structure 200m in which distances between impedance electrodes are different from one another, according to an embodiment of the present disclosure. Referring to FIG. 17, in the biosensor electrode structure 200m, impedance electrodes 121, 122, 127, and 128 for calculating bioimpedances in different regions are spaced apart from one another. For example, a seventh impedance electrode 127 and an eighth impedance electrode 128 are sequentially disposed at the working electrode 111 in the direction of the second impedance electrode 122. Distances d1, d2, and d3 between the impedance electrodes gradually increase from the working electrode 111 to the eighth impedance electrode 128. For example, the distance d2 between the second impedance electrode 122 and the seventh impedance electrode 127 is greater than the distance d1 between the first impedance electrode 121 and the second impedance electrode 122. The distance d3 between the seventh impedance electrode 127 and the eighth impedance electrode 128 is greater than the distance d2 between the second impedance electrode 122 and the seventh impedance electrode 127.

The distances d1, d2, and d3 between the impedance electrodes are associated with depths 11, 12, and 13 of the subject 10 in which the bioimpedances are calculated. For example, as the distance between the impedance electrodes increases, the depth of the subject 10 in which the bioimpedances are calculated increases. By changing the distance between the impedance electrodes, the bioimpedance is calculated at different depths in the subject 10, which may be used to check an extent to which a target material spreads from blood vessels toward the skin.

In addition, the enzyme is activated by predicting a time point at which the target material spreads to the first region 11 and operating the working electrode 111. Information about the target material may be determined by using a change in the bioimpedance for the first region 11 of the subject 10.

FIG. 18 illustrates a method of providing an electrical stimulation and detecting an electrical response in a biosensor, according to an embodiment of the present disclosure. Referring to FIG. 18, the first stimulator 132 of the biosensor 100 provides the first electrical stimulation to the subject 10 through the impedance electrode part 120 at step S1810. The first stimulator 132 provides a first electrical stimulation through the first and second impedance electrodes 121 and 122 or provides a first electrical stimulation through the third and fourth impedance electrodes 123 and 124 that are different from the first and second impedance electrodes 121 and 122. In this case, the first and second impedance electrodes 121 and 122 are disposed between the third and fourth impedance electrodes 123 and 124. The first electrical stimulation includes at least one of an AC voltage and an AC current. Therefore, the first stimulator is implemented by a current source or a voltage source.

The first detector 142 detects the first electrical response corresponding to the first electrical stimulation from the subject 10 through the impedance electrode part 120 at step S1820. The first detector 142 detects the first electrical response through the first and second impedance electrodes 121 and 122. When the first stimulator 132 provides the first electrical stimulation to the subject 10, a current path is generated in the subject 10. Therefore, the first detector 142 detects the first electrical response, such as current from the current path.

The second stimulator 134 provides the second electrical stimulation to enzyme of the working electrode 111 at step S1830. The enzyme responds to the second electrical stimulation to cause the target material that is a specific material in the subject 10 to react, to change the electrolytic component of the subject 10. The change in the electrolytic component changes the first electrical response to the second electrical response.

The first detector 142 detects the second electrical response corresponding to the first electrical stimulation and the second electrical stimulation from the subject 10 at step S1840.

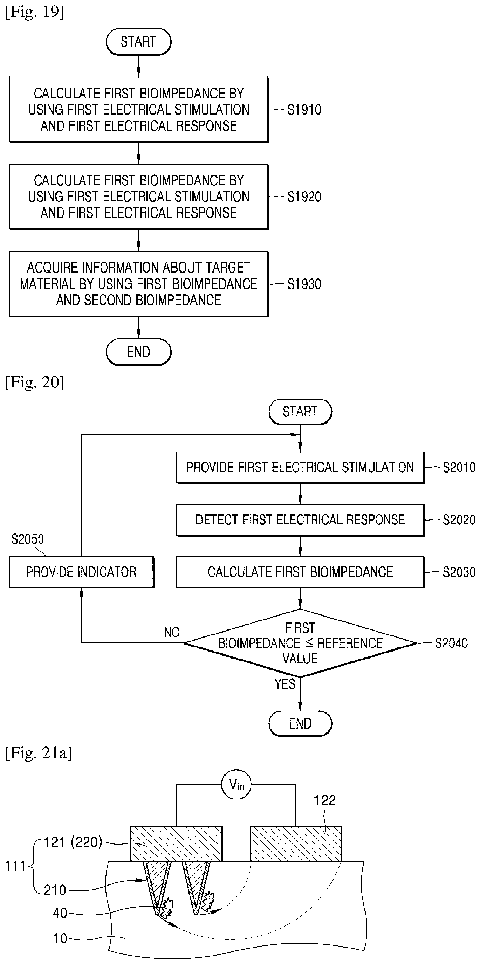

FIG. 19 illustrates a method of acquiring information about a target material in the biosensor 100, according to an embodiment of the present disclosure. Referring to FIG. 19, the calculator 150 of the biosensor 100 calculates a first bioimpedance by using the first electrical stimulation and the first electrical response at step S1910. When the first electrical stimulation is voltage and the first electrical response is current, the calculator 150 calculates the first bioimpedance by using a complex ratio of the first electrical stimulation to the first electrical response. Alternatively, the calculator 150 calculates the real part of the complex ratio of the first electrical stimulation to the first electrical response as the first bioimpedance. A noise component of the subject 10 is reduced when the real part is calculated as the first bioimpedance, but the present embodiment is not limited thereto. That is, both the real part and the imaginary part of the complex ratio of the first electrical stimulation to the first electrical response may be used when calculating the bioimpedance.

In addition, the calculator 150 calculates the second bioimpedance by using the first electrical stimulation and the second electrical stimulation at step S1920. The second electrical stimulation is current. When reactant by reaction of the enzyme includes electrons, the second electrical response is greater than the first electrical response. Alternatively, the calculator 150 calculates the second bioimpedance by using the complex ratio of the first electrical stimulation to the second electrical response, or the real part of the complex ratio. Therefore, the second bioimpedance may be less than the first bioimpedance.

The controller 160 acquires information about the target material by using the first bioimpedance and the second bioimpedanceat step S1930. For example, when the change amount of the first bioimpedance and the second bioimpedance is equal to or greater than a reference value, the controller 160 determines that the target material exists. When the change amount increases with reference to time, the controller 160 determines that the concentration of the target material increases. When the change amount decreases with reference to time, the controller 160 determines that the concentration of the target material decreases. In addition, the controller 160 determines whether the needle electrode is sufficiently inserted into the subject based on the change in the bioimpedance.

FIG. 20 illustrates a method of operating a biosensor, according to another embodiment of the present disclosure. Hereinafter, for convenience of description, it is assumed that the needle electrode 310 is disposed at the first and second impedance electrodes 121 and 122.

Referring to FIG. 20, the first stimulator 132 of the biosensor 100 provides the first electrical stimulation to the subject 10 through the impedance electrode part 120 at step S2010. The stimulator 132 provides a first electrical stimulation through the first and second impedance electrodes 121 and 122 or provides a first electrical stimulation through the third and fourth impedance electrodes 123 and 124 that are different from the first and second impedance electrodes 121 and 122. In this case, the first and second impedance electrodes 121 and 122 are disposed between the third and fourth impedance electrodes 123 and 124. The first electrical stimulation includes at least one of AC voltage and AC current. Therefore, the first stimulator is implemented by a current source or a voltage source.

The first detector 142 detects the first electrical response corresponding to the first electrical stimulation from the subject 10 through the impedance electrode part 120 at step S2020. The first detector 142 detects the first electrical response through the first and second impedance electrodes 121 and 122. A current path is generated in the subject 10 when the first stimulator 132 provides the first electrical stimulation to the subject 10. Therefore, the first detector 142 detects the first electrical response, such as current from the current path.

The calculator 150 of the biosensor 100 calculates a first bioimpedance by using the first electrical stimulation and the first electrical response at step S2030. When the first electrical stimulation is voltage and the first electrical response is current, the calculator 150 calculates the first bioimpedance by using a complex ratio of the first electrical stimulation to the first electrical response. Alternatively, the calculator 150 calculates a real part of the complex ratio of the first electrical stimulation to the first electrical response as the first bioimpedance. When the real part is calculated as the first bioimpedance, a noise component of the subject 10 is reduced, but the present embodiment is not limited thereto. That is, when calculating the bioimpedance, both the real part and the imaginary part of the complex ratio of the first electrical stimulation to the first electrical response may be used.

The controller 160 determines whether the first bioimpedance is less than a reference value at step S2040. The first bioimpedance when the needle electrode is exposed to air varies from the first bioimpedance when the needle electrode is inserted into the subject. For example, when the needle electrode is inserted through skin of the subject, the first bioimpedance considerably decreases. Therefore, the controller 160 determines whether the needle electrode is inserted into the subject by using a value of the first bioimpedance. For example, when the first bioimpedance is less than the reference value, the controller 160 determines that the needle electrode is inserted into the subject. In this case, the reference value is a general value when the needle electrode is inserted into the subject and may be previously defined by examination.

When the first bioimpedance is less than the reference value at step S2040, the process ends

When the first bioimpedance is greater than or equal to the reference value at step S2040, the controller 160 determines that the needle electrode 310 is not inserted into the subject, and an indicator is provided at step S2050, indicating that the needle electrode 310 is not inserted into the subject. The indicator is provided through sound, text, or an image, for example. A user may check the indicator and manipulate the biosensor 100 such that the needle electrode is inserted into the subject.

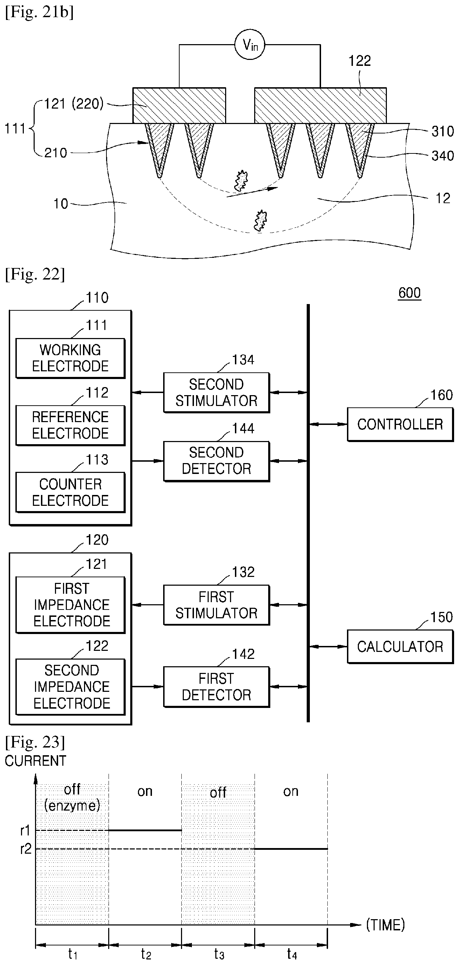

FIGS. 21a and 21b are reference diagrams for describing a method of removing foreign material adsorbed onto the biosensor electrode structure. Referring to FIG. 21a, when the enzyme electrode 210 is maintained in a state of penetrating the subject 10, foreign material such as immune substances or proteins is adsorbed onto the enzyme electrode 210. Since the foreign material 40 may inhibit the enzyme to react with the target material, it may be difficult to acquire accurate information about the target material.

According to an embodiment of the present disclosure, the second stimulator 134 provides the third electrical stimulation such that a non-uniform electric field is formed between the enzyme electrode 210 and the second impedance electrode 122. The third electrical stimulation is an electrical stimulation for removing the foreign material 40 adsorbed onto the enzyme electrode 210. For example, the second stimulator 134 provides AC voltage or AC current as the third electrical stimulation. Therefore, a dense electric field is formed in a sharp region of the enzyme electrode 210 by a lightning effect. The non-uniform electric field induces a dielectrophoresis force and the foreign material 40 is separated from the enzyme electrode 210 by the dielectrophoresis force.

As described above, when the foreign material 40 is adsorbed onto the electrode, the performance of the biosensor electrode structure is maintained by separating the foreign material from the enzyme electrode 210 by the dielectrophoresis force, instead of detecting the adsorption and performing correction.

Referring to FIG. 21b, the needle electrode 310 is disposed at the second impedance electrode 122. A decomposition layer 340 including an enzyme that reacts with the foreign material is coated on the surface of the needle electrode 310. The enzyme included in the decomposition layer 340 decomposes the foreign material or produces a useful material to the human body by synthesis with the foreign material. For example, the decomposition layer 340 includes lipase enzyme that decomposes fat. Therefore, fat moved to the decomposition layer 340 is decomposed by the dielectrophoresis force. Alternatively, the decomposition layer 340 includes catalase capable of removing reactive oxygen species, glutathione peroxidase, or carboxyl enzyme capable of decomposing protein. Therefore, the foreign material existing in a region 12, in which the bioimpedance is measured, is removed by the dielectrophoresis force. The biosensor performs a function of calculating the amperometric of the subject 10 in addition to calculation of the bioimpedance.

FIG. 22 is a block diagram of a biosensor 600 having a current calculation function, according to an embodiment of the present disclosure. The biosensor 600 capable of performing both calculation of a bioimpedance and calculation of a current will be referred to as a hybrid-type sensor. Referring to FIG. 22, the working electrode part 110 of the biosensor 600 includes a working electrode 111, a reference electrode 112, and a counter electrode 113. The working electrode 111 penetrates s subject 10 and includes an enzyme that reacts with a specific material in the subject 10. The reference electrode 112 is the reference of the working electrode 111. The counter electrode 113 is used to measure a current in the subject 10.

The biosensor 600 includes a first stimulator 132, a second stimulator 134, a first detector 142, a second detector 144, and a calculator 150. The first stimulator 132 provides a first electrical stimulation to an impedance electrode part 120. The second stimulator 134 provides a second electrical stimulation to the working electrode 111. The first detector 142 detects first and second electrical responses of the subject 10 through the impedance electrode part 120. The second detector 144 detects a third electrical response corresponding to the second electrical stimulation from the counter electrode 113. The calculator 150 calculates a bioimpedance by using the first electrical stimulation, the first electrical response, and the second electrical stimulation. The biosensor 100 further includes a controller 160 that acquires information about a target material based on the detected third electrical response and the calculated bioimpedance.

In this case, the first electrical stimulation is an AC voltage or AC, and the second electrical stimulation is a DC voltage or DC. The first electrical response is a current path formed in the subject 10 by the first electrical stimulation, and the second electrical response is a value to which the first electrical response is changed by activation of the enzyme. The third electrical response is a current path formed by activation of the enzyme.

The detection of the third electrical response by activation of the enzyme is used to acquire information about the target material. For example, the second stimulator 134 applies the second electrical response to the working electrode 111, and the second detector 144 detects the third electrical response from the subject 10. When the detected third electrical response is less than a reference value, the controller 160 determines that the target material is absent. In addition, the controller 160 determines whether the target material is increased or reduced by using a change in the third electrical response with reference to time.

As described above, when a foreign material is adsorbed onto the working electrode 111, the enzyme does not cause the target material to react. Even in this case, the detected third electrical response is reduced, but the third electrical response is not generated by a change in the concentration of the target material.

FIG. 23 illustrates an example of a third electrical response with reference to time, according to an embodiment of the rpesent disclosure. Referring to FIG. 23, the third electrical response is not detected during time intervals t1 and t3 in which the second electrical stimulation is not applied to the working electrode 111. The third electrical responses r1 and r2 are detected during time intervals t2 and t4 in which the second electrical stimulation is applied to the working electrode 111. The third electrical response I2 during the fourth time interval t4 is less than the third electrical response I1 during the second time interval t2.

As descried above, the concentration of the target material in the subject 10 may decrease, the enzyme may not react with the target material because the foreign material is adsorbed onto the working electrode 111, the enzyme may be damaged, or a target material content in the subject 10 may change. Therefore, the biosensor may have difficulty in acquiring information about the target material due to reduction in the third electrical response.

However, the hybrid-type biosensor 600 according to the present embodiment more accurately acquires information about the target material by calculating the bioimpedance.

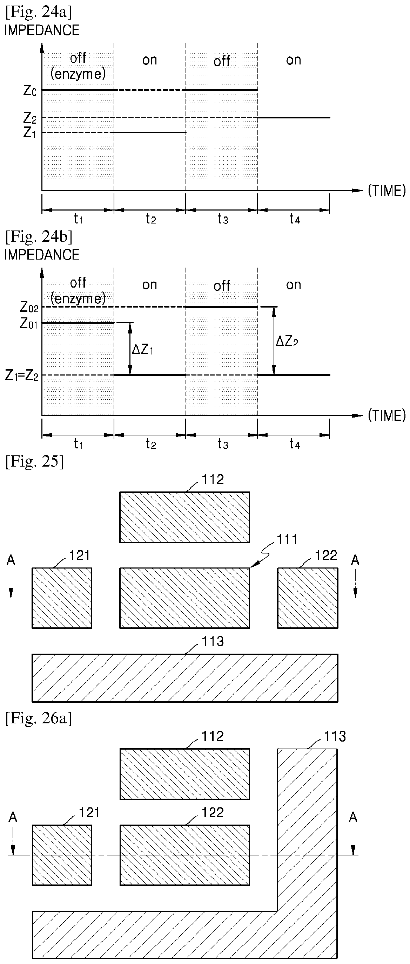

FIGS. 24a and 24b illustrate examples of first and second electrical responses with reference to time, according to an embodiment of the present disclsoure. Referring to FIG. 24a, the first bioimpedance Z0 is substantially the same during the time intervals t1 and t3 in which the second electrical stimulation is not applied to the working electrode 111. The second bioimpedance increases during the time intervals t2 and t4 in which the second electrical stimulation is applied to the working electrode 111. For example, the second bioimpedance Z2 during the fourth time interval t4 is greater than the second bioimpedance Z1 during the second time interval t2. In this case, the biosensor 100 determines that the bioimpedance is substantially unchanged when enzyme is deactivated, and determines that the target material is changed when the enzyme is activated based on the increase in the second bioimpedance.

Referring to FIG. 24b, the first bioimpedance Z02, which is calculated during the third time interval t3 in which the second electrical stimulation is not applied to the working electrode 111, is greater than the first bioimpedance Z01, which is calculated during the first time interval t1 in which the second electrical stimulation is not applied to the working electrode 111. However, the second bioimpedance Z2, which is calculated during the fourth time interval t4 in which the second electrical stimulation is applied to the working electrode 111, is substantially equal to the second bioimpedance Z1, which is calculated during the second time interval t2 in which the second electrical stimulation is applied to the working electrode 111. A change amount ΔZ2 in the bioimpedance during the third time interval t3 and the fourth time interval t4 is greater than a change amount ΔZ1 in the bioimpedance between the first time interval t1 and the second time interval t2. Therefore, the biosensor determines that an internal environment of the subject is changed, foreign material is adsorbed onto the electrode, or the electrode is damaged, based on the changes in the bioimpedance (ΔZ1 and ΔZ2), and determines that the concentration of the target material increases based when the second bioimpedance does not change.

The quantitative analysis for the target material may require further data, such as information about an electrode state or the environment. Acquisition of information about the target material using the bioimpedance further reflects the electrode state and change in environment of the subject, compared to acquisition of information about the target material using current.

When the information about the target material is acquired by using the bioimpedance, it may be necessary to alternately provide the first electrical stimulation and the second electrical stimulation and to calculate the bioimpedance, thus increasing a load, as compared to current measurement.

Therefore, the hybrid-type biosensor 600 determines information about the target material by using a current and, when the detected third electrical response is reduced, determines information about the target material by using the bioimpedance.

In addition, the hybrid-type biosensor 600 finally determines information about the target material by combining the information about the target material using a current with the information about the target material using the bioimpedance. For example, the hybrid-type biosensor 600 determines the average of the information about the target material using a current and the information about the target material using the bioimpedance, as final information about the target material.

FIG. 25 is a plan view of an electrode structure applicable to the hybrid-type biosensor 600 in FIG. 22. Referring to FIG. 25, in the electrode structure applicable to the hybrid-type biosensor 600, the first and second impedance electrodes 121 and 122 are spaced apart from each other with the working electrode 111 disposed therebetween, and the reference electrode 112 and the counter electrode 113 are spaced apart from each other with the working electrode 111 disposed therebetween. The first and second impedance electrodes 121 and 122, the counter electrode 113, and the reference electrode 112 surround the working electrode 111 while being spaced apart from the working electrode 111. The first working electrode 111, the counter electrode 113, the second working electrode 111, and the reference electrode 112 are arranged in a clockwise or counterclockwise direction.

Since the cross-section taken along A-A in FIG. 25 is the same as the cross-section taken along A-A in FIG. 2a, a detailed description will be omitted. The working electrode 111 includes a plurality of enzyme electrodes 210, in which case the enzyme electrodes 210 are arranged in a one-dimensional or two-dimensional manner. At least one of the first and second impedance electrodes 121 and 122 may or may not include one or more needle electrodes 310, and the first and second impedance electrodes 121 and 122 are disposed to be symmetrical to the working electrode 111.

The reference electrode 112 and the counter electrode 113 are spaced apart from each other with the working electrode 111 disposed therebetween. The width of the reference electrode 112 is substantially equal to or different from the width of the working electrode 111. Since the counter electrode 113 detects current, the cross-section of the electrode 113 is larger than that of the working electrode 111. For example, the counter electrode 113 is disposed to correspond to at least a partial region of the first and second impedance electrodes 121 and 122 while being disposed to correspond to the working electrode 111.

One or more needle electrodes 310 are further disposed in at least one of the reference electrode 112, the counter electrode 113, and the first and second impedance electrodes 121 and 122, and have a size that corresponds to the size of the enzyme electrode 210 of the working electrode 111. The needle electrodes 310 are arranged to be symmetrical to the working electrode 111. For example, the needle electrode 310 is disposed at the first and second impedance electrodes 121 and 122 or is disposed at the reference electrode 112 and the counter electrode 113.

The reference electrode 112 is illustrated in FIG. 25, but the present disclosure is not limited thereto, and one of the first impedance electrode 121 and the second impedance electrode 122 may serve as the reference electrode 112. In this case, the reference electrode 112 is not separately provided.

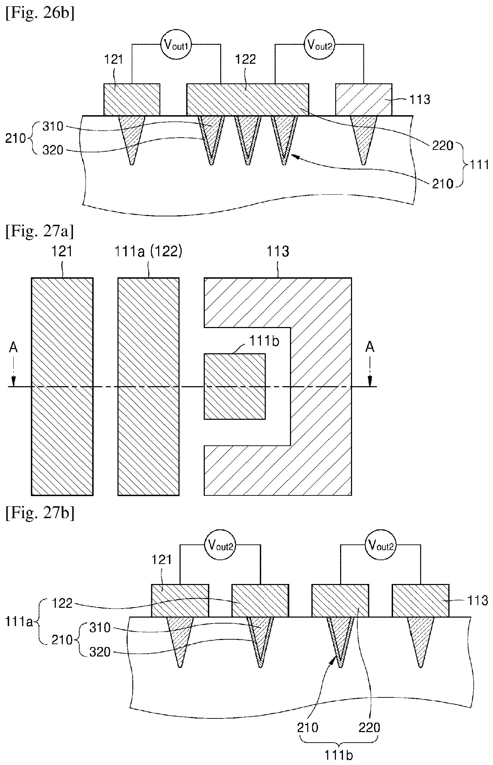

FIG. 26a is a plan view of an electrode structure applicable to the hybrid-type biosensor 600 in FIG. 22, according to another embodiment of the present disclosure, and FIG. 26b is a cross-sectional view of the electrode structure in FIG. 26a. Referring to FIGs. 27a and 27b, the enzyme electrode 210 is disposed at the second impedance electrode 122. Therefore, the second impedance electrode 122 and the enzyme electrode 210 constitute the working electrode 111. The enzyme electrode 210 is disposed at the second impedance electrode 122, simplifying the electrode structure.

The counter electrode 113 is spaced apart from the working electrode 111 and is disposed to correspond to the working electrode 111 and to the first impedance electrode 121 and at least a partial region of the reference electrode 112. Therefore, a cross-sectional size of the counter electrode 113 increases, enhancing detection strength. When the first impedance electrode 121 serves as the reference electrode 112, the reference electrode 112 is not separately provided.

FIG. 27a is a plan view of an electrode structure applicable to the hybrid-type biosensor 600 in FIG. 22, according to another embodiment of the present disclosure, and FIG. 27b is a cross-sectional view of the electrode structure in FIG. 27a.

Referring to FIGs. 27a and 27b, the enzyme electrode 210 is disposed at the second impedance electrode 122. Therefore, the second impedance electrode 122 and the enzyme electrode 210 constitute the first working electrode 111a. The hybrid-type biosensor 600 calculates a bioimpedance by using the first impedance electrode 121 and the first working electrode 111a.

The hybrid-type biosensor 600 further includes the second working electrode 111b and the counter electrode 113. The second working electrode 111b includes a support electrode 220 and the enzyme electrode 210. The first working electrode 111a is used to calculate the bioimpedance, whereas the second working electrode 111b is used to calculate current. The second working electrode 111b is surrounded by the counter electrode 113. A reference electrode may be separately provided, but the counter electrode 113 serves as the reference electrode.

As described above, the working electrode part 110 may be divided into the first and second working electrodes 111a and 111b, reducing signal interference caused by sharing of the working electrode.

As illustrated in FIGS. 15 to 17, the hybrid-type biosensor 600 further includes an impedance electrode, such as third to eighth impedance electrodes, for calculating a bioimpedance in another region. A distance between impedance electrodes varies depending on a region in which a bioimpedance is to be calculated and a depth thereof.

The enzyme has been described as being used to measure a bioimpedance, but the present disclosure is not limited thereto, and antibodies may be used in addition to enzyme. The antibodies vary depending on a type of a target material to be detected.

It should be understood that embodiments described herein should be considered in a descriptive sense only and not for purposes of limitation. Descriptions of features or aspects within each embodiment should typically be considered as available for other similar features or aspects in other embodiments.

## Claims

1. A biosensor electrode structure comprising:
first and second impedance electrodes (121, 122) that are spaced apart from each other, wherein the first and second impedance electrodes are configured to contact a subject (10), and a first stimulator (132) configured to provide a first electrical stimulation to the subject through the first and second impedance electrodes;
a working electrode (111) configured to penetrate the subject and a second stimulator (134) configured to provide a second electrical stimulation to the subject through the working electrode, the working electrode including an enzyme disposed on at least a partial surface of the working electrode, the enzyme being
configured to cause a target material present in the subject to react to change a first electrical response corresponding to the first electrical stimulation applied to the subject to a second electrical response in the subject, different than the first electrical response, when the enzyme is activated by the second electrical stimulation; and
wherein first and second impedance electrodes are configured to receive the first electrical response when the enzyme is deactivated and receive the second electrical response from the subject when the enzyme is activated,
wherein activation of the enzyme is reversible by stopping the second electrical stimulation.

2. The biosensor electrode structure of claim 1, wherein the working electrode includes at least one enzyme electrode having a sharp, needle-shaped end configured to penetrate the subject, and at least a partial surface portion in which the enzyme is provided.

3. The biosensor electrode structure of claim 2, further comprising a protection layer that is configured to cover a surface of the enzyme electrode and includes a biodegradable material.

4. The biosensor electrode structure of claim 2, wherein the working electrode is spaced apart from and is disposed between the first and second impedance electrodes.

5. The biosensor electrode structure of claim 2, wherein the working electrode further includes a support electrode having a plate shape and configured to contact an end of the enzyme electrode opposite the needle-shaped end.

6. The biosensor electrode structure of claim 2, wherein the end of the enzyme electrode opposite the needle-shaped end is configured to contact at least one of the first and second impedance electrodes.

7. The biosensor electrode structure of claim 1, further comprising a needle electrode having a sharp, needle- shaped end configured to penetrate the subject, and an end opposite the needle-shaped end that is configured to contact at least one of the first and second impedance electrodes.

8. The biosensor electrode structure of claim 1, further comprising a third impedance electrode configured to contact the subject and that is spaced apart from the second impedance electrode.

9. The biosensor electrode structure of claim 8, wherein a distance between the first impedance electrode and the second impedance electrode is different from a distance between the second impedance electrode and the third impedance electrode.

10. A method of operating a biosensor, the biosensor including a working electrode configured to penetrate a subject only up to the subcutaneous layer, the working electrode including an enzyme disposed on at least a partial surface of the working electrode, the enzyme being configured to cause a target material present in the subject to react to change a first electrical response corresponding to a first electrical stimulation applied to the subject to a second electrical response in the subject, different than the first electrical response, when the enzyme is activated, and a plurality of impedance electrodes that contact the subject and are spaced apart from each other, the method comprising:
providing using a first stimulator (132) the first electrical stimulation to the subject through the plurality of impedance electrodes;
detecting the first electrical response corresponding to the first electrical stimulation from the subject through the plurality of impedance electrodes when the enzyme is deactivated;
providing using a second stimulator (134) a second electrical stimulation to the subject through the working electrode wherein the second electrical stimulation activates the enzyme; and
detecting the second electrical response corresponding to the first electrical stimulation and the second electrical stimulation from the subject through the plurality of impedance electrodes when the enzyme is activated,
wherein activation of the enzyme is reversible by stopping the second electrical stimulation.

11. The method of claim 10, further comprising:
calculating a first bioimpedance by using the first electrical stimulation and the first electrical response; and
calculating a second bioimpedance by using the first electrical stimulation and the second electrical response.

12. The method of claim 11, further comprising determining information about the target material by using a change amount of the first bioimpedance and the second bioimpedance.

## Patentansprüche

1. Biosensor-Elektrodenstruktur, umfassend:
eine erste und zweite Impedanzelektrode (121, 122), die voneinander beabstandet sind, wobei die erste und zweite Impedanzelektrode dazu konfiguriert sind, ein Subjekt (10) zu berühren, und
einen ersten Stimulator (132), der dazu konfiguriert ist, dem Subjekt durch die erste und zweite Impedanzelektrode eine erste elektrische Stimulation bereitzustellen;
eine Arbeitselektrode (111), die dazu konfiguriert ist, in das Subjekt einzudringen, und einen zweiten Stimulator (134), der dazu konfiguriert ist, dem Subjekt durch die Arbeitselektrode eine zweite elektrische Stimulation bereitzustellen,
wobei die Arbeitselektrode ein Enzym beinhaltet, das auf mindestens einer Teilfläche der Arbeitselektrode angeordnet ist, wobei das Enzym dazu konfiguriert ist, zu bewirken, dass ein Zielmaterial, das in dem Subjekt vorhanden ist, reagiert, um eine erste elektrische Antwort zu ändern, die der
ersten elektrischen Stimulation entspricht, die an das Subjekt angelegt ist, zu einer zweiten elektrischen Antwort in dem Subjekt, die sich von der ersten elektrischen Antwort unterscheidet,
wenn das Enzym durch die zweite elektrische Stimulation aktiviert wird; und
wobei erste und zweite Impedanzelektroden dazu konfiguriert sind,
die erste elektrische Antwort zu empfangen, wenn das Enzym deaktiviert ist, und die zweite elektrische Antwort von dem Subjekt zu empfangen, wenn das Enzym aktiviert ist,
wobei ein Aktivieren des Enzyms reversibel ist, indem die zweite elektrische Stimulation gestoppt wird.

2. Biosensor-Elektrodenstruktur nach Anspruch 1, wobei die Arbeitselektrode mindestens eine Enzymelektrode beinhaltet, die ein scharfes, nadelförmiges Ende, das dazu konfiguriert ist, in das Subjekt einzudringen, und mindestens einen Teilflächenabschnitt aufweist, in dem das Enzym bereitgestellt ist.

3. Biosensor-Elektrodenstruktur nach Anspruch 2, ferner umfassend eine Schutzschicht, die dazu konfiguriert ist, dass sie eine Fläche der Enzymelektrode bedeckt, und ein biologisch abbaubares Material beinhaltet.

4. Biosensor-Elektrodenstruktur nach Anspruch 2, wobei die Arbeitselektrode von der ersten und der zweiten Impedanzelektrode beabstandet und zwischen diesen angeordnet ist.

5. Biosensor-Elektrodenstruktur nach Anspruch 2, wobei die Arbeitselektrode ferner eine Trägerelektrode beinhaltet, die eine Plattenform aufweist und dazu konfiguriert ist, ein Ende der Enzymelektrode gegenüber dem nadelförmigen Ende zu berühren.

6. Biosensor-Elektrodenstruktur nach Anspruch 2, wobei das Ende der Enzymelektrode, das dem nadelförmigen Ende gegenüberliegt, dazu konfiguriert ist, mindestens eine der ersten und zweiten Impedanzelektroden zu berühren.

7. Biosensor-Elektrodenstruktur nach Anspruch 1, ferner umfassend eine Nadelelektrode, die ein scharfes, nadelförmiges Ende, das dazu konfiguriert ist, in das Subjekt einzudringen, und ein Ende aufweist, das gegenüber dem nadelförmigen Ende liegt, das dazu konfiguriert ist, mindestens eine der ersten und zweiten Impedanzelektrode zu berühren.

8. Biosensor-Elektrodenstruktur nach Anspruch 1, ferner umfassend eine dritte Impedanzelektrode, die dazu konfiguriert ist, das Subjekt zu berühren, und die von der zweiten Impedanzelektrode beabstandet ist.

9. Biosensor-Elektrodenstruktur nach Anspruch 8, wobei sich eine Distanz zwischen der ersten Impedanzelektrode und der zweiten Impedanzelektrode von einer Distanz zwischen der zweiten Impedanzelektrode und der dritten Impedanzelektrode unterscheidet.

10. Verfahren zum Betreiben eines Biosensors, wobei der Biosensor eine Arbeitselektrode umfasst, die dazu konfiguriert ist, nur bis zu der subkutanen Schicht in das Subjekt einzudringen, wobei die Arbeitselektrode ein Enzym beinhaltet, das auf mindestens einer Teilfläche der Arbeitselektrode angeordnet ist, wobei das Enzym dazu konfiguriert ist, zu bewirken, dass ein Zielmaterial, das
in dem Subjekt vorhanden ist, reagiert, um eine erste elektrische Antwort, die einer ersten elektrischen Stimulation entspricht, die auf das Subjekt angewendet wird, auf eine zweite elektrische Antwort in dem Subjekt zu reagieren, die sich von der ersten elektrischen Antwort unterscheidet, wenn das Enzym aktiviert ist, und eine Vielzahl von Impedanzelektroden, die das Subjekt berühren und voneinander beabstandet sind, wobei das Verfahren Folgendes umfasst:
Bereitstellen der ersten elektrischen Stimulation an das Subjekt unter Verwendung eines ersten Stimulators (132) durch die Vielzahl von Impedanzelektroden;
Erfassen der ersten elektrischen Antwort, die der ersten elektrischen Stimulation von dem Subjekt entspricht, durch die Vielzahl von Impedanzelektroden, wenn das Enzym deaktiviert ist;
Bereitstellen einer zweiten elektrischen Stimulation an das Subjekt unter Verwendung eines zweiten Stimulators (134)
durch die Arbeitselektrode, wobei die zweite elektrische Stimulation das Enzym aktiviert; und
Erfassen der zweiten elektrischen Antwort, die der ersten elektrischen Stimulation und der zweiten elektrischen Stimulation entspricht, von dem Subjekt durch die Vielzahl von Impedanzelektroden,
wenn das Enzym aktiviert ist,
wobei ein Aktivieren des Enzyms reversibel ist, indem die zweite elektrische Stimulation gestoppt wird.

11. Verfahren nach Anspruch 10, ferner umfassend:
Berechnen einer ersten Bioimpedanz unter Verwendung der ersten elektrischen Stimulation und der ersten elektrischen Antwort; und
Berechnen einer zweiten Bioimpedanz unter Verwendung der ersten elektrischen Stimulation und der zweiten elektrischen Antwort.

12. Verfahren nach Anspruch 11, ferner umfassend ein Bestimmen von Informationen über das Zielmaterial unter Verwendung eines Änderungsbetrags der ersten Bioimpedanz und der zweiten Bioimpedanz.

## Revendications

1. Structure d'électrode de capteur biologique comprenant :
des première et deuxième électrodes d'impédance (121, 122) qui sont espacées l'une de l'autre, lesdites première et deuxième électrodes d'impédance étant conçues pour entrer en contact avec un sujet (10), et un premier stimulateur (132) conçu pour fournir une première stimulation électrique au sujet par l'intermédiaire des première et deuxième électrodes d'impédance ;
une électrode de travail (111) conçue pour pénétrer dans le sujet et un second stimulateur (134) conçu pour fournir une seconde stimulation électrique au sujet par l'intermédiaire de l'électrode de travail,
l'électrode de travail comprenant une enzyme disposée sur au moins une surface partielle de l'électrode de travail, l'enzyme étant conçue pour amener une matière cible présente dans le sujet à réagir pour changer une première réponse électrique correspondant à la première stimulation électrique appliquée au sujet en une seconde réponse électrique chez le sujet, différente de la première réponse électrique, lorsque l'enzyme est activée par la seconde stimulation électrique ; et
des première et deuxième électrodes d'impédance étant conçues pour recevoir la première réponse électrique lorsque l'enzyme est désactivée et recevoir la seconde réponse électrique en provenance du sujet lorsque l'enzyme est activée,
ladite activation de l'enzyme étant réversible en arrêtant la seconde stimulation électrique.

2. Structure d'électrode de capteur biologique selon la revendication 1, ladite électrode de travail comprenant au moins une électrode à enzyme possédant une extrémité pointue en forme d'aiguille conçue pour pénétrer dans le sujet, et au moins une partie de surface partielle dans laquelle l'enzyme est fournie.

3. Structure d'électrode de capteur biologique selon la revendication 2, comprenant en outre une couche de protection qui est conçue pour recouvrir une surface de l'électrode à enzyme et comprenant une matière biodégradable.

4. Structure d'électrode de capteur biologique selon la revendication 2, ladite électrode de travail étant espacée des première et deuxième électrodes d'impédance et étant disposée entre elles.

5. Structure d'électrode de capteur biologique selon la revendication 2, ladite électrode de travail comprenant en outre une électrode de support possédant une forme de plaque et conçue pour entrer en contact avec une extrémité de l'électrode à enzyme opposée à l'extrémité en forme d'aiguille.

6. Structure d'électrode de capteur biologique selon la revendication 2, ladite extrémité de l'électrode à enzyme opposée à l'extrémité en forme d'aiguille étant conçue pour entrer en contact avec au moins l'une des première et deuxième électrodes d'impédance.

7. Structure d'électrode de capteur biologique selon la revendication 1, comprenant en outre une électrode aiguille possédant une extrémité pointue en forme d'aiguille conçue pour pénétrer dans le sujet, et une extrémité opposée à l'extrémité en forme d'aiguille qui est conçue pour entrer en contact avec au moins l'une des première et deuxième électrodes d'impédance.

8. Structure d'électrode de capteur biologique selon la revendication 1, comprenant en outre une troisième électrode d'impédance conçue pour entrer en contact avec le sujet et qui est espacée de la deuxième électrode d'impédance.

9. Structure d'électrode de capteur biologique selon la revendication 8, une distance entre la première électrode d'impédance et la seconde électrode d'impédance étant différente d'une distance entre la seconde électrode d'impédance et la troisième électrode d'impédance.

10. Procédé de fonctionnement d'un capteur biologique, le capteur biologique comprenant une électrode de travail conçue pour pénétrer dans un sujet uniquement jusqu'à la couche sous-cutanée, l'électrode de travail comprenant une enzyme disposée sur au moins une surface partielle de l'électrode de travail, l'enzyme étant conçue pour amener une matière cible présente chez le sujet à réagir pour changer une première réponse électrique correspondant à une première stimulation électrique appliquée au sujet en une seconde réponse électrique chez le sujet, différente de la première réponse électrique, lorsque l'enzyme est activée, et une pluralité d'électrodes d'impédance qui contactent le sujet et sont espacées l'une de l'autre, le procédé comprenant :
la fourniture, à l'aide d'un premier stimulateur (132), de la première stimulation électrique au sujet par l'intermédiaire de la pluralité d'électrodes d'impédance ;
la détection de la première réponse électrique correspondant à la première stimulation électrique provenant du sujet par l'intermédiaire de la pluralité d'électrodes d'impédance lorsque l'enzyme est désactivée ;
la fourniture à l'aide d'un second stimulateur (134) d'une
seconde stimulation électrique au sujet par l'intermédiaire de l'électrode de travail, ladite seconde stimulation électrique activant l'enzyme ; et
la détection de la seconde réponse électrique correspondant à la première stimulation électrique et
de la seconde stimulation électrique provenant du sujet par l'intermédiaire de la pluralité d'électrodes d'impédance lorsque l'enzyme est activée,
ladite activation de l'enzyme étant réversible en arrêtant la seconde stimulation électrique.

11. Procédé selon la revendication 10, comprenant en outre :
le calcul d'une première impédance bioélectrique à l'aide de la première stimulation électrique et
de la première réponse électrique ; et
le calcul d'une seconde impédance bioélectrique à l'aide de la première stimulation électrique et
de la seconde réponse électrique.

12. Procédé selon la revendication 11, comprenant en outre la détermination d'informations concernant la matière cible à l'aide d'une quantité de changement de la première impédance bioélectrique et de la seconde impédance bioélectrique.
